# EUROPÄISCHE PATENTANMELDUNG

(11) **EP 1 518 536 A2**
(43) Veröffentlichungstag der Anmeldung: **30.03.2005**
(21) Anmeldenummer: 04018427.7
(22) Anmeldetag: 04.08.2004
(51) Int. Cl.: A61K 7/06

(54) **Mittel für die oxidative Haarbehandlung und Verfahren zur dauerhaften Haarverformung**

(30) Priorität: 26.09.2003 DE 10344881
(71) Anmelder: Wella Aktiengesellschaft, 64274 Darmstadt (DE)
(72) Erfinder: Arlt, Thilo Dr., 8112 Otelfingen (CH); Bitterli, Natalie, 3280 Murten (CH); Clément, Pierre-Alain, 1752 Villars-sur-Glane (CH)

(57) **Zusammenfassung**

Mittel zur oxidativen Haarbehandlung dadurch gekennzeichnet, das es Azodicarboxamid oder ein Azodicarboxylat, dessen Derivat und/oder Salz, vorzugsweise in wässriger, wäßrig-alkoholischer oder alkoholischer Zubereitung enthält. Beansprucht wird ferner ein Verfahren zur dauerhaften Haarverformung, bei dem man das Haar bevor und/oder nachdem man es in die gewünschte Form bringt, mit einem keratinreduzierenden Verformungsmittel behandelt, danach spült, sodann mit einem Fixiermittel oxidativ nachbehandelt und erneut spült, dadurch gekennzeichnet, daß man als Fixiermittel ein vorstehend beschriebenes Mittel verwendet.

Das so behandelte Haar besitzt eine gleichmäßige und haltbare Umformung und zeigt keine Aufhellungen.

## Beschreibung

Die vorliegende Erfindung betrifft ein Mittel in dem Azodicarboxamide oder Azodicarboxylate oder deren Derivate vorzugsweise in wässriger, wässrig-alkoholischer oder alkoholischer Lösungen vorliegen, zur Durchführung der oxidativen Nachbehandlung bei der reduktiven Veränderung von Keratinfasern, insbesondere der dauerhaften Haarverformung.

Bei der reduktiven Veränderung von Keratinfasern wird das Haar zunächst mit einem Verformungsmittel, welches eine Öffnung der Disulfidbindungen des Haarkeratins bewirkt, behandelt und sodann in die gewünschte Form gebracht. Als Verformungsmittel werden hierbei in der Regel keratinreduzierende Mercaptoverbindungen, wie zum Beispiel Salze oder Ester von Mercaptocarbonsäuren, oder Sulfite verwendet. Anschließend wird das Haar mit Wasser oder einem geeigneten Zwischenbehandlungsmittel gespült. Sodann werden die reduzierten Haarfasern mit einem Fixiermittel oxidativ nachbehandelt. Hierbei werden Disulfidbindungen innerhalb des Haarkeratins geschlossen, welche für die dauerhafte Haltbarkeit der Haarverformung, insbesondere beim Wellen oder Glätten, maßgebend sind.

Bei Fixiermitteln auf der Basis von Wasserstoffperoxid, Peroxidsalzen (Perborate, persulfate etc.) oder Bromaten wird ein Teil der Disulfid- und Thiolgruppen des Haarkeratins zu höheren Oxidationsstufen des Schwefels, insbesondere zu Cysteinsäure aufoxidiert. Hierdurch wird das Haarkeratin irreversibel geschädigt. Im Falle peroxidhaltiger Fixiermittel werden außerdem die Farbpigmente des Haares (Melanine) partiell zerstört, was insbesondere bei asiatischem Haar mit einer Aufhellung des Haares verbunden ist.

Es stellte sich die Aufgabe, die bei der dauerhaften Haarverformung auftretenden Nachteile bezüglich Bleichwirkung und Cysteinsäurebildung zu vermeiden, ohne hierdurch die Haarstruktur zu beeinträchtigen.

Es wurde gefunden, daß die gestellte Aufgabe in hervorragender Weise durch ein Mittel für die oxidativen Haarbehandlung gemäß Anspruch 1 gelöst wird.

Besonders bevorzugt ist das Mittel für die oxidativen Haarbehandlung ein Mittel für die Durchführung der oxidativen Nachbehandlung (Fixierung) von zuvor reduktiv behandelten Haaren bei der dauerhaften Haarverformung. Ein solches Haardauerverformungsfixiermittel wird nachfolgend mit "Fixiermittel" bezeichnet.

Vorzugsweise liegt das Mittel für die Durchführung der oxidativen Haarbehandlung als wässrige oder wässrig-alkoholische oder alkoholische Zubereitung vor.

Für das erfindungsgemäße Mittel besonders geeignete Oxidationsmittel sind Azocarboxamide der allgemeinen Formel (I) oder Azodicarboxylate der allgemeinen Formel (II) 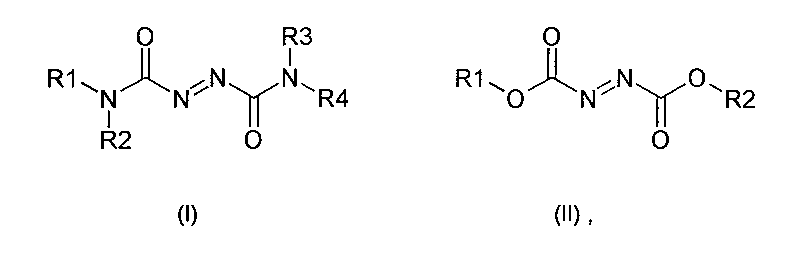 wobei R1, R2, R3 und R4 unabhängig voneinander geradkettiges oder verzweigtes C₁- bis C₄-Alkyl, geradkettiges oder verzweigtes C₁- bis C₄-Hydroxyalkyl mit 1 bis 3 Hydroxylgruppen, Phenyl oder Benzyl bedeuten.

Besonders bevorzugt sind die Substituenten R1, R2, R3 und R4 unabhängig voneinander Methyl, Ethyl, Propyl, Isopropyl, Benzyl oder Phenyl. Ganz besonders bevorzugt sind die Substituenten R1, R2, R3 und R4 gleich und bedeuten Methyl oder Ethyl.

Die Azocarboxamide können jedoch auch am Amid-Stickstoff unter Einbeziehung des Stickstoffs gemeinsam mit einer Alkylengruppe einen Heterocyclus bilden. Als Beispiele seien die Verbindungen Azodicarboxyldipiperide der Formel (III) und Azodicarboxyldimorpholide der Formel (IV) genannt.

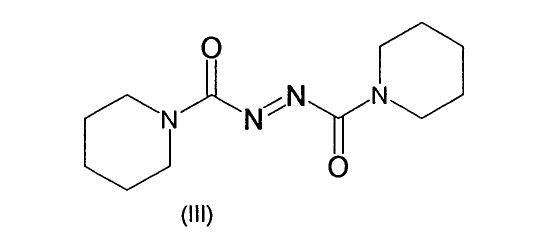

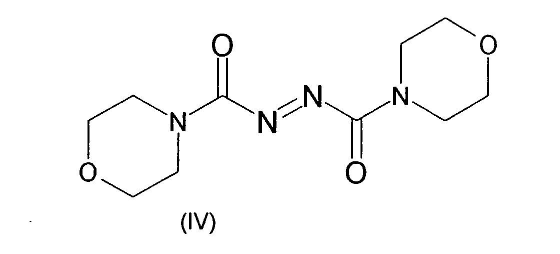

Das Mittel für die Durchführung der oxidativen Haarbehandlung enthält günstiger weise 0,1 bis 20 Gewichtsprozent, vorzugsweise 0,5 bis 15 Gewichtsprozent, besonders bevorzugt 1 bis 10 Gewichtsprozent mindestens eines Azodicarboxamids, Azodicarboxylats, dessen Salzes oder dessen Derivats.

Gegebenenfalls kann das Mittel für die Durchführung der oxidativen Haarbehandlung zusätzlich übliche Oxidationsmittel wie z. B. Wasserstoffperoxid, Peroxidsalze oder Natriumbromat enthalten. Vorzugsweise enthalten die Mittel jedoch das Azodicarboxamid, Azodicarboxylat, dessen Salz und/oder dessen Derivat als einziges Oxidationsmittel.

Der pH-Wert des gebrauchsfertigen Mittels für die Durchführung der oxidativen Haarbehandlung liegt im allgemeinen in einem Bereich von 1,5 bis 10, vorzugsweise 2 bis 8 besonders bevorzugt 25 bis 75 Die Finstellung (des pH-Wertes erfolgt mit üblichen Alkalisierungsmitteln und puffernden Stoffen wie zum Beispiel Ammoniak, Alkalihydroxide, Alkalicarbonate, Alkalihydrogencarbonate, Citratpuffer, Phosphorsäure und deren Salze, Zitronensäure und deren Salze und Ascorbinsäure und deren Salze.

Das Mittel für die Durchführung der oxidativen Haarbehandlung kann beispielsweise in Form einer wäßrigen, wäßrig-alkoholischen, alkoholischen Lösung oder einer Emulsion aber auch in verdickter Form auf wäßriger Basis, insbesondere als Creme, Gel oder Paste vorliegen. Vorzugsweise liegt das erfindungsgemäße Mittel als wässrige oder wäßrig-alkoholische Lösung vor.

Neben dem Azodicarboxamid, Azodicarboxylat, dessen Salz oder dessen Derivat kann das Mittel für die Durchführung der oxidativen Haarbehandlung noch Zusätze enthalten, wie sie in haarkosmetischen Zubereitungen üblich sind. Hierzu gehören Quell- und Penetrationsstoffe, wie zum Beispiel Harnstoff, 2-Pyrrolidon, 1-Methyl-2-pyrrolidon und Dipropylenglykolmonomethylether, sowie Säuerungsmittel beispielsweise aromatische Sulfonsäuren, Salzsäure, Schwefelsäure, Phosphorsäure, Pyrophosphorsäuren oder Polyphosphorsäuren, saure Salze starker Säuren, Ascorbinsäure, Oxalsäure, Malonsäure, Benzoesäure, Salicylsäure, Zitronensäure und Gerbsäuren.

Weiterhin können in dem Mittel für die Durchführung der oxidativen Haarbehandlung Netzmittel und Emulgatoren aus der Gruppe der anionischen, nichtionischen, kationischen und amphoteren oder zwitterionischen oberflächenaktiven Agenzien enthalten sein. Geeignete oberflächenaktive Agenzien sind insbesondere
a) anionische oberflächenaktive Agenzien, wie beispielsweise Alkali-, Erdalkali-, Ammonium- oder Alkanolaminsalze von Alkylsulfonaten, Alkylsulfaten und Alkylethersulfaten, wie zum Beispiel Natriumlaurylalkoholdiglykolethersulfat, Natrium- oder Triethanolaminsalze von Alkylsulfaten mit 12 bis 18, vorzugsweise 12 bis 14 Kohlenstoffatomen, die Natrium- oder Triethanolaminsalze von Lauryl- oder Tetradecylethersulfaten, das Dinatriumsalz des Sulfosuccinhalbesters von Alkanolamiden, Seifen und Polyethercarbonsäuren;
b) nichtionische oberflächenaktive Agenzien, wie beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl- und Stearylalkohol, allein oder im Gemisch, oxethylierte Lanolinalkohole, oxethyliertes Lanolin, oxethylierte Alkylphenole mit 8 bis 30 Kohlenstoffatomen im Alkylrest und 1 bis 10 Ethylenoxideinheiten im Molekül, Fettsäurealkanolamide sowie oxethylierte Sorbitanfettsäureester;
c) kationische oberflächenaktive Agenzien, wie beispielsweise Dilauryldimethylammoniumchlorid, Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder - bromid, Alkyldimethylhydroxyethylammoniumchloride oder -bromide, Dialkyldimethylammoniumchloride oder -bromide, Alkylpyridiniumsalze, beispielsweise Lauryl- oder Cetylpyridiniumchlorid, Alkylamidoethyltrimethylammoniumethersulfate, Verbindungen mit kationischem Charakter wie Aminoxide, beispielsweise Alkyldimethylaminoxide oder Alkylaminoethyldimethylaminoxide und
d) amphotere oder zwitterionische oberflächenaktive Agenzien wie beispielsweise Carboxylderivate des Imidazols, N-Alkyl- und N-Alkylamidobetaine, N-Alkylsulfobetaine, N-Alkylaminopropionate, Alkyldimethylcarboxymethylammoniumsalze mit 12 bis 18 Kohlenstoffatomen sowie Fettsäurealkylamidobetaine, beispielsweise Fettsäureamidopropyldimethylaminoessigsäurebetain.

Selbstverständlich kann das Mittel für die Durchführung der oxidativen Haarbehandlung alle für derartige Mittel üblichen Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, ferner Farbstoffe, Trübungsmittel, wie zum Beispiel Polyethylenglykolester, Lösungsmittel wie Alkohole, wie beispielsweise Ethanol, Propanol und Isopropanol; Lösungsvermittler wie Glycerin, Ethylenglykol und Propylenglykol; Puffersubstanzen, Parfümöle, haarkonditionierende oder haarpflegende Bestandteile, wie zum Beispiel Kämmbarkeitsverbesserer und gegen Haarflattern (hair fizz) wirkende Stoffe wie kationische Polymere, z. B. CTFA: POLYQUATERNIUM-1,
CTFA: POLYQUATERNIUM-4, CTFA: POLYQUATERNIUM-5,
CTFA: POLYQUATERNIUM-6, CTFA: POLYQUATERNIUM-7,
CTFA: POLYQUATERNIUM-10, CTFA: POLYQUATERNIUM-11,
CTFA: POLYQUATERNIUM-16, CTFA: POLYQUATERNIUM-22,
CTFA: POLYQUATERNIUM-32, CTFA: POLYQUATERNIUM-35,
CTFA: POLYQUATERNIUM-36, CTFA: POLYQUATERNIUM-37,
CTFA: POLYQUATERNIUM-39, CTFA: POLYQUATERNIUM-44,
CTFA: POLYQUATERNIUM-45, CTFA: POLYQUATERNIUM-46,
CTFA: POLYQUATERNIUM-47, CTFA: POLYSILICONE-3,
CTFA: POLYSILICONE-4, CTFA: POLYSILICONE-5, CTFA: POLYSILICONE-6,
CTFA: POLYSILICONE-7 CTFA: POLYSILICONE-8 und CTFA: POLYSILICONE-13; Lanolinderivate, Cholesterin, Pantothensäure, Proteinderivate und Proteinhydrolysate, Betain, Provitamine und Vitamine sowie Pflanzenextrakte enthalten. Die Abkürzung "CTFA" steht für *International Cosmetic Ingredient Dictionary and Handbook,* Eighth Edition 2000 (ISBN 1-882621-22-0).

Die Bestandteile der kosmetischen Zubereitung werden zur Herstellung des Mittels für die Durchführung der oxidativen Haarbehandlung in für diesen Zweck üblichen Mengen eingesetzt. Beispielsweise werden Netzmittel und Emulgatoren in Konzentrationen von 0,2 bis 30 Gewichtsprozent, Alkohole in einer Konzentration von 1 bis 80 Gew.%, haarkonditionierende oder haarpflegende Bestandteile in einer Konzentration von 0,1 bis 10 Gew.%, und Verdickungsmittel in Konzentrationen von 0,1 bis 25 Gewichtsprozent bezogen auf das Mittel eingesetzt.

Die Anwendungstemperatur des gebrauchsfertigen Mittels für die Durchführung der oxidativen Haarbehandlung liegt in einem Bereich von 10°C bis 60°C, vorzugsweise 20°C bis 55°C, besonders bevorzugt 20°C bis 40°C. Die Einwirkungszeiten betragen von 1 bis 45 Minuten, vorzugsweise 2 bis 25 Minuten, besonders bevorzugt 3 bis 15 Minuten.

Ein weiterer Gegenstand der vorliegenden Erfindung ist ein Verfahren zur dauerhaften Haarverformung, bei dem man das Haar bevor und/oder nachdem man es in die gewünschte Form bringt mit einem keratinreduzierenden Verformungsmittel behandelt, spült, sodann mit einem Fixiermittel oxidativ nachbehandelt, spült, anschließend zur Frisur legt und sodann trocknet, welches dadurch gekennzeichnet ist, daß für die oxidative Nachbehandlung als Fixiermittel das vorstehend beschriebene Mittel für die Durchführung der oxidativen Haarbehandlung verwendet wird. Die Spülung erfolgt vorzugsweise mit Wasser.

Bei einer besonderen Ausführungsform des erfindungsgemäßen Verfahrens wird das Haar zunächst mit dem keratinreduzierenden Verformungsmittel behandelt, das Verformungsmittel nach der Einwirkungszeit ausgespült, anschließend wird das Haar mit dem vorstehend beschriebenen Fixiermittel auf der Basis eines Azodicarboxamids oder Azodicarboxylats, deren Derivate oder Salze als Oxidationsmittel behandelt (vorfixiert) und sodann mit einem Fixiermittel auf der Basis von Wasserstoffperoxid oder Bromat nachbehandelt (nachfixiert). Besonders vorteilhaft weist das Fixiermittel für die Nachfixierung eine geringere Konzentration an Oxidationsmittel auf als für solche Mittel üblich; so beträgt vorzugsweise die Konzentration an Wasserstoffperoxid nur 0,1 bis 1 Gew.% bzw. an Bromat nur 1 bis 5 Gew.%.

Bei dem erfindungsgemäßen Verfahren wird das Haar gewaschen, mit einem Handtuch frottiert, gegebenenfalls mit einem Teil des keratinreduzierenden Verformungsmittels vorgefeuchtet, in einzelne Strähnen aufgeteilt und auf Wickler gewickelt. Der Durchmesser der Wickler beträgt hierbei, je nachdem ob eine Dauerwellung oder eine Entkräuselung der Haare gewünscht wird, entweder etwa 5 bis 13 Millimeter oder etwa 15 bis 35 Millimeter. Auf das gewickelte Haar wird anschließend eine für die dauerhafte Haarverformung ausreichende Menge eines Verformungsmittels aufgetragen. Die für die dauerhafte Haarverformung erforderliche Gesamtmenge des Verformungsmittels beträgt im allgemeinen etwa 80 bis 120 Gramm.

Die bei dem erfindungsgemäßen Verfahren verwendbaren Verformungsmittel enthalten übliche keratinreduzierende Verbindungen, wie zum Beispiel bestimmte Mercaptoverbindungen, insbesondere Thioglykolsäure oder deren Salze, Thiomilchsäure oder deren Salze, Cystein, Cysteamin sowie Salze oder Ester von Mercaptocarbonsäuren. Diese Verformungsmittel enthalten die keratinreduzierenden Verbindungen in den für derartige Mittel üblichen Mengen, beispielsweise die Ammoniumsalze der Thioglykol- oder Thiomilchsäure in einer Menge von etwa 2 bis 12 Gewichtsprozent. Der pH-Wert dieser Verformungsmittel beträgt im allgemeinen etwa 7 bis 11, wobei die Einstellung des pH-Wertes vorzugsweise mit Ammoniak, Monoethanolamin, Ammoniumcarbonat oder Ammoniumhydrogencarbonat erfolgt. Bei sauer (zum Beispiel auf pH = 6,5 bis 6,9) eingestellten Verformungsmitteln werden vorzugsweise Ester von Mercaptocarbonsäuren, wie beispielsweise Monothioglykolsäureglykolester oder Monothioglykolsäureglycerinester oder Sulfite, in einer Konzentration von etwa 2 bis 25 Gewichtsprozent verwendet.

Die Verformungsmittel können weiterhin alle für derartige Mittel üblichen Zusatzstoffe, beispielsweise Quell- und Penetrationsstoffe, Verdickungsmittel, Netzmittel und Emulgatoren, Alkohole, Lösungsvermittler, Stabilisatoren, Farbstoffe, Parfümöle sowie haarkonditionierende oder haarpflegende Bestandteile, enthalten. Die vorstehend genannten Zusatzstoffe werden in den für solche Zwecke üblichen Mengen verwendet, zum Beispiel die Netzmittel und Emulgatoren in Konzentrationen von etwa 0,2 bis 30 Gewichtsprozent, während die Verdickungsmittel in einer Menge von etwa 0,1 bis 25 Gewichtsprozent in dem Verformungsmittel enthalten sein können.

Das bei dem erfindungsgemäßen Verfahren verwendete Verformungsmittel kann sowohl in Form einer wäßrigen Lösung oder Emulsion als auch in verdickter Form auf wäßriger Basis, insbesondere als Creme, Gel oder Paste, oder in Form eines Aerosolschaums vorliegen.

Nach einer für die dauerhafte Haarverformung ausreichenden Einwirkungszeit, welche je nach der Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und sodann mit etwa 50 Gramm bis 350 Gramm, vorzugsweise 80 bis 200 Gramm des vorstehend beschriebenen gebrauchsfertigen Fixiermittels oxidativ nachbehandelt.

Nach einer Einwirkungszeit des Fixiermittels von 1 bis 45 Minuten, vorzugsweise 3 bis 25 Minuten, besonders bevorzugt 5 bis 15 Minuten werden die Wickler entfernt und das abgewickelte Haar, nur falls erforderlich, nochmals mit dem Fixiermittel oxidativ nachbehandelt. Sodann wird das Haar, vorzugsweise mit Wasser, gespült, zur Frisur gelegt und getrocknet.

Das so behandelte Haar besitzt eine gleichmäßige und haltbare Umformung. Im Unterschied zu Haaren, die mit Peroxid fixiert wurden und die insbesondere auf japanischen Haaren eine deutlich feststellbare Farbverschiebung in Richtung rot und gelb aufweisen (Aufhellungsgrad), liegen die Werte bei dem erfindungsgemäßen Fixiermittel im Bereich unbehandelter Haarsträhnen.

### Beispiele

Die nachfolgenden Beispiele seien zur genauen Beschreibung des erfindungsgemäßen Verfahrens aufgeführt.

In diesen Beispielen wurde das Haar jeweils in folgender Weise reduktiv vorbehandelt:

Je 3 bis 4 unbehandelte und damit nicht vorgeschädigte Zählhaarsträhnen (bestehend entweder aus je 100 mittelbraunen, europäischen Haaren oder aus je 60 schwarzen, asiatischen Haaren jeweils mit einer Länge von genau 16,5 Zentimetern) wurden naß auf genormte Spiralwickler (Innendurchmesser: 3 Millimeter) aufgewickelt und nach dem Konditionieren im Exsikkator (Temperatur 20°C; Luftfeuchte: 99%) mit einer handelsüblichen Welllösung (Thioglykolsäuregehalt: 10,8 Gewichtsprozent; pH = 8,2) behandelt. Die Auftragemenge an Wellflüssigkeit wurde über das Verhältnis 1 : 1,2 errechnet (1g Haar: 1,2 ml Wellflüssigkeit). Die Menge von 1,2 ml auf etwa 1 Gramm Haar entspricht einer Menge von 50 ml Wellösung pro Damenkopf mit einem Durchschnittsgewicht von ca. 30g Haar pro Kopf.

Als Einwirkungszeit wurden 15 Minuten gewählt; die Einwirkungstemperatur des Reduktionsmittels betrug 45 Grad Celsius.

### Beispiel 1

Nach dem Ende der Einwirkungszeit wird die überschüssige Welllösung mit Wasser ausgespült und sodann die folgende Fixierlösung aufgetragen.

| | |
|---|---|
| 10,0 g | Diisopropylazodicarboxylat |
| 45,0 g | Isopropanol |
| 45,0 g | Wasser |
| 100,0 g | |

Der pH-Wert dieser Fixierlösung liegt bei 6,0.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt einen guten Allgemeinzustand. Es ist nicht aufgehellt.

### Beispiel 2

Analog zu Beispiel 1 wird nach dem Ende der Einwirkungszeit die überschüssige Welllösung mit Wasser ausgespült und sodann die folgende Fixierlösung aufgetragen.

| | |
|---|---|
| 10,0 g | Diisopropylazodicarboxylat |
| 44,2 g | Isopropanol |
| 44,2 g | Wasser |
| 1,0 g | Laurylethersulfat (4EO) |
| 0,6 q | kationisches Polymer (CTFA: POLYQUATERNIUM-35) |
| 100,0 g | |

Der pH-Wert dieser Fixierlösung liegt bei 5,3.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit. Es ist nicht aufgehellt.

### Beispiel 3

Analog zu Beispiel 1 wird nach dem Ende der Einwirkungszeit die überschüssige Welllösung mit Wasser ausgespült und sodann die folgende Fixierlösung aufgetragen.

| | |
|---|---|
| 10,0 g | Azodicarbonsäuredibenzylester |
| 90,0 q | Isopropanol |
| 100,0 g | |

Der pH-Wert dieser Fixierlösung liegt bei 5.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, Es ist nicht aufgehellt.

### Beispiel 4

Analog zu Beispiel 1 wird nach dem Ende der Einwirkungszeit die überschüssige Welllösung mit Wasser ausgespült und sodann die folgende Fixierlösung aufgetragen.

| | |
|---|---|
| 2,00 g | N,N,N',N'-Tetramethyldicarboxamid |
| 98,00 q | Wasser |
| 100,00 g | |

Der pH-Wert dieser Fixierlösung liegt bei 5,2.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, ist nicht aufgehellt.

### Beispiel 5

Analog zu Beispiel 1 wird nach dem Ende der Einwirkungszeit die überschüssige Welllösung mit Wasser ausgespült und sodann die folgende Fixierlösung aufgetragen.

| | |
|---|---|
| 2,5 g | Azodicarboxyl-dimorpholid |
| 97,5 g | Wasser |
| 100,0 g | |

Der pH-Wert dieser Fixierlösung liegt bei 7,0.

Das Haar wird 10 Minuten lang bei 22 Grad Celsius fixiert und anschließend mit Wasser gespült. Sodann werden die Wickler entfernt und das Haar mit lauwarmem Wasser ausgespült. Schließlich wird das Haar zur Frisur gelegt und sodann getrocknet.

Das so behandelte Haar zeigt eine gute Umformung und Haltbarkeit, ist nicht aufgehellt.

## Patentansprüche

1. Mittel zur oxidativen Haarbehandlung, **dadurch gekennzeichnet, dass** es als Oxidationsmittel mindestens ein Azodicarboxamid, Azodicarboxylat, dessen Salz und/oder dessen Derivat enthält.

2. Mittel nach Anspruch 1, **dadurch gekennzeichnet, dass** es als wässrige, wässrig-alkoholische oder alkoholische Zubereitung vorliegt.

3. Mittel nach Anspruch 1 oder 2, **dadurch gekennzeichnet, daß** das Azodicarboxamid, Azodicarboxylat und/oder deren Derivat ausgewählt ist aus N,N,N',N'-Tetramethyldicarboxamid, Diisopropylazodicarboxylat, Azodicarbonsäuredibenzylester und Azodicarboxyl-dimorpholid.

4. Mittel nach einem der Ansprüche 1 bis 3, **dadurch gekennzeichnet, daß** das Azodicarboxamid, Azodicarboxylat, dessen Salz und/oder dessen Derivat in einer Menge von 1 bis 10 Gewichtsprozent enthalten ist.

5. Mittel nach einem der Ansprüche 1 bis 4, **dadurch gekennzeichnet, daß** es einen pH-Wert von 2,5 bis 8,0 aufweist.

6. Mittel nach einem der Ansprüche 1 bis 5, **dadurch gekennzeichnet, dass** es ein Haardauerverformungsfixiermittel ist.

7. Mittel nach einem der Ansprüche 1 bis 6, **dadurch gekennzeichnet, dass** es mindestens einen kosmetischen Zusatzstoff enthält, der ausgewählt ist aus der Gruppe Säuerungsmittel, Netzmittel, Emulgatoren, Verdickungsmittel, Farbstoffe, Trübungsmittel, Alkohole, Lösungsvermittler, Puffersubstanzen, Parfümöle, Kämmbarkeisverbesserer, haarkonditionierende Stoffe, haarpflegende Stoffe, gegen Haarflattern (hair fizz) wirkende Stoffe, kationische Polymere, Vitamine, Provitamine und Pflanzenextrakte.

8. Mittel nach Anspruch 7, **dadurch gekennzeichnet, dass** das kationische Polymer ausgewählt ist aus CTFA: POLYQUATERNIUM-1, CTFA: POLYQUATERNIUM-4, CTFA: POLYQUATERNIUM-5, CTFA: POLYQUATERNIUM-6, CTFA: POLYQUATERNIUM-7, CTFA: POLYQUATERNIUM-10, CTFA: POLYQUATERNIUM-11, CTFA: POLYQUATERNIUM-16, CTFA: POLYQUATERNIUM-22, CTFA: POLYQUATERNIUM-32, CTFA: POLYQUATERNIUM-35, CTFA: POLYQUATERNIUM-36, CTFA: POLYQUATERNIUM-37, CTFA: POLYQUATERNIUM-39, CTFA: POLYQUATERNIUM-44, CTFA: POLYQUATERNIUM-45, CTFA: POLYQUATERNIUM-46, CTFA: POLYQUATERNIUM-47, CTFA: POLYSILICONE-3, CTFA: POLYSILICONE-4, CTFA: POLYSILICONE-5, CTFA: POLYSILICONE-6, CTFA: POLYSILICONE-7, CTFA: POLYSILICONE-8 und CTFA: POLYSILICONE-13.

9. Verfahren zur dauerhaften Haarverformung, bei dem man das Haar bevor und/oder nachdem man es in die gewünschte Form bringt, mit einem keratinreduzierenden Verformungsmittel behandelt, danach spült, sodann mit einem Fixiermittel oxidativ nachbehandelt und erneut spült, **dadurch gekennzeichnet, daß** man als Fixiermittel ein Mittel nach einem der Ansprüche 1 bis 8 verwendet.

10. Verfahren nach Anspruch 9, **dadurch gekennzeichnet, daß** man das Fixiermittel bei einer Temperatur von 20°C bis 50°C verwendet.
